(19) Europäisches Patentamt
European Patent Office
Office européen des brevets

(11) **EP 4 541 415 B1**

(12) **EUROPEAN PATENT SPECIFICATION**

(45) Date of publication and mention
of the grant of the patent:
**04.03.2026 Bulletin 2026/10**

(21) Application number: **23383061.1**

(22) Date of filing: **18.10.2023**

(51) International Patent Classification (IPC):
**A61N 1/36** *(2006.01)*       **A61N 1/40** *(2006.01)*
**A61N 1/04** *(2006.01)*       A61N 1/05 *(2006.01)*

(52) Cooperative Patent Classification (CPC):
**A61N 1/36002; A61N 1/0476; A61N 1/36034;
A61N 1/40;** A61N 1/0484; A61N 1/0526;
A61N 1/36146

(54) **DEVICE FOR TREATING TUMORS USING PULSED ALTERNATING CURRENTS**

VORRICHTUNG ZUR BEHANDLUNG VON TUMOREN MIT GEPULSTEN WECHSELSTRÖMEN

DISPOSITIF DE TRAITEMENT DE TUMEURS PAR COURANTS ALTERNATIFS PULSÉS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**23.04.2025 Bulletin 2025/17**

(73) Proprietor: **Universitat Pompeu Fabra
08002 Barcelona (ES)**

(72) Inventor: **IVORRA CANO, Antoni
08002 Barcelona (ES)**

(74) Representative: **TRBL Intellectual Property
Plaza de Castilla 3, 7°A
28046 Madrid (ES)**

(56) References cited:
**US-A1- 2005 209 641      US-A1- 2023 173 288
US-B2- 8 718 756**

**Description**

**FIELD OF THE INVENTION**

The present invention belongs to the field of medical technologies. More specifically, the invention relates to a device suitable for selectively eradicating and/or for slowing down the progression of tumors based on the application of pulsed AC fields of moderate amplitude over an extended period of time in a region of a body's subject. This allows mild electroporation to take place in tumor cells, enabling a sufficient calcium uptake as to eradicate them without harming surrounding healthy tissue. The invention also relates to a method comprising the operation of a device according to any of the embodiments described in this document.

**BACKGROUND OF THE INVENTION**

In the field of treatment of tumors, such as those related to cancer, there are cases in which tumors are either inoperable or do not respond sufficiently to conventional therapies (e.g., chemotherapy, radiotherapy, or immunotherapy). Therefore, the treatment of said cases requires the use of alternative or complementary therapies.

The delivery of alternating electrical currents to produce heating in order to thermally destroy tumors (thermal ablation) or to increase the effectiveness of pharmacological or radiotherapy treatments by mild warming (adjuvant hyperthermia) has been a clinical reality for decades. More recently, since the 1990s, electroporation-based therapies have emerged.

Electroporation (EP) is the phenomenon by which cell membrane permeability to ions and molecules is non-selectively increased when the cell is exposed to a high-intensity electric field. This effect is typically achieved by applying, by means of electrodes placed strategically around the tumor, short high-voltage DC pulses or high-voltage pulsed alternating waveforms.

Electroporation can be classified as either reversible or irreversible. In reversible electroporation, the cells remain viable after a transient stage of increased cell membrane permeability. However, in irreversible electroporation (IRE), which takes places typically under certain conditions such as very large field magnitudes, membrane permeabilization is permanent and the process leads to cell lysis. Irreversible electroporation may also refer to the situation in which membrane permeabilization is temporary but sufficient to cause the corresponding cell to die.

Electroporation can be applied in vivo and may be used, among other therapeutical applications such as gene transfection, in the treatment of tumors. In fact, several electroporation-based therapies have been developed for the treatment of tumors in the past years. These include:

- Electrochemotherapy (ECT): the tissue to be treated is briefly subjected to a high-intensity electric field in order to enhance the penetration into cells of a chemotherapeutic chemical agent (e.g., bleomycin or cisplatin). The electric field exposure is delivered through needle electrodes, and it consists of a short sequence of square pulses with a magnitude in the order of hundreds of V/cm and a duration in the order of 100 $\mu$s. The electric field exposure is intended to produce reversible electroporation to prevent that healthy cells are damaged by the treatment.
- Calcium electroporation (CaEP): understood as a subclass of ECT in which, instead of using chemotherapeutic chemical agents, calcium solutions are employed. Cell death is induced after electroporation because supraphysiological concentrations of calcium are forced into the cells. Remarkably, tumor cells are substantially more sensitive to calcium than healthy cells.
- Irreversible electroporation (IRE): the field exposure is designed so that thermal damage is limited and the cells are killed primarily by disruption of cell homeostasis by excessive cell membrane permeabilization. No chemical agents are employed. As in the case of ECT and CaEP the electric field exposure is delivered through needle electrodes, and it consists of a short sequence of square pulses with a duration in the order of 100 $\mu$s. In this case, however, the magnitudes are substantially higher, in the order of one or two kV/cm.
- High-Frequency Irreversible Electroporation (HFIRE): pulsed alternating fields are applied to minimize unsought electrostimulation. Typically, the alternating fields consist of bipolar pulses with a length of less than 10 $\mu$s applied in bursts. The field magnitudes are typically well above two kV/cm.
- Nanosecond Pulsed Field Ablation (nsPFA): Instead of using pulses in the order of microseconds, in nsPFA pulse lengths are in the order of nanoseconds. In this case, the field magnitudes are well above ten kV/cm and many more pulses are applied than in the case of conventional IRE or HFIRE. This treatment is longer than conventional IRE or HFIRE. Total treatment times range from seconds to minutes.

The delivery of low DC currents for minutes is also a known technique used as a therapy for tumor ablation. Non-specific tissue ablation is achieved by the electrochemical reactions generated at the electrodes and the migration of the generated chemical species. This treatment is known as Electrochemical Treatment (EChT).

All the above referred treatments, except for adjuvant hyperthermia, are focal therapies. That is, these therapies selectively destroy tumors (and not healthy tissue) because they restrict (i.e., focalize) the energy delivery to the specific location of the tumor. As such, these treatments cannot be used to simultaneously treat multiple tumors dispersed in a region of the body. In addition, these treatments are short in time: the electric current is applied for seconds or minutes in EChT and in thermal ablation, and for fractions of a second in electroporation-based treatments.

It must be noted that current electroporation-based therapies apply relatively high electric field magnitudes (much higher than 100 V/cm) and, in order to prevent thermal damage by ohmic heating, use short exposures (shorter than 10 ms). In fact, electroporation is frequently depicted as a phenomenon that cannot be observed for lower field magnitudes, even if the exposure is very long. However, such a depiction is the result of a simplification due to practical constraints. Long exposures are avoided in electroporation studies to avoid excessive heat and electrochemical reactions. Furthermore, electroporation studies are typically focused only on observing increases of membrane permeabilities which are sufficient to allow the transit of large molecules, neglecting the transit of small ions. Thus, knowledge regarding the electroporation effects for low field magnitudes and long exposures is very limited. The state-of-the-art lacks an electroporation-based treatment that may be performed with moderately low amplitude fields that leads to a membrane permeability large enough to permit the uptake of ions such as calcium, which, as explained above, may induce tumor cell death.

Radiotherapy and chemotherapy, which are the most common non-surgical cancer treatment therapies, are intended to target rapidly proliferating cells, which are characteristic of cancerous tumors. The main reason for this focus is that cancer cells divide and grow at a much faster rate than healthy cells in the body. Targeting these proliferating cells helps to slow down or halt the growth of the tumor, ultimately leading to the destruction of the cancer cells.

The cell cycle is the series of events that a cell undergoes as it grows, replicates its DNA, and divides into two daughter cells. It consists of several phases that exhibit varying degrees of sensitivity to stress. Cells are generally most sensitive to stresses, such as radiation, during the phases in which they are actively dividing. Because of this, long-course therapies are extended over long periods, often spanning several weeks. This extended duration provides the opportunity to impact on cancer cells during different phases of the cell cycle, maximizing the overall effectiveness of the treatment.

Within this spirit, so-called Tumor Treatment Fields (TTFields), with frequencies in the order of 100 kHz and magnitudes in the order of a very few V/cm, are applied continuously for weeks and months in a region of the body in order to hamper the growth of tumors. TTFields' mechanism of action is thought to be related to anti cell division effects related to the polarization of intracellular structures during mitosis. The very low amplitudes employed in these treatments, deliberately limited to prevent excessive tissue heating, lead to low effectiveness in tumor cell eradication, since the effects of TTFields are counteracted by regulatory or repair mechanisms of the cells. While long-course therapies, such as those involving the use of TTfields in the art, target cancer cells during different phases of the cell cycle, if applied intermittently, they can also provide healthy tissues with time to repair and recover between doses. This helps minimize the risk of severe damage to healthy tissues and organs. Nevertheless, it is of tantamount importance to correctly establish the time-pattern of the treatment, i.e., the duration and frequency of the sessions thereof. This is necessary in order to balance the time of exposure (which, if long, is more effective for cancer cell damage but also damages healthy tissue), and the frequency of the sessions (sufficiently long to ensure healthy cell recovery but not as long as to allow tumor cells to recover).

US2005/209641 discloses a device fir selectively eradicating tumors.

In summary, although treatments based on electrical currents have improved cancer care by complementing conventional treatments (e.g., chemotherapy, radiotherapy, or immunotherapy), the current state of the art still presents certain limitations. Mainly, the proposed focal treatments are highly invasive and only suitable to treat a small number of localized tumors. In addition, in all proposed non-thermal electrical treatments, including TTFields, which is a non-focal modality, the magnitude of the field is limited to avoid overheating of the tissue, thus reducing the effectiveness of these treatments.

The present invention is aimed to remedy these drawbacks, through a novel device for eradicating or slowing down the progression of tumors based on the delivery of pulsed AC electric fields to a region of a patient's body comprising one or more tumors.

## BRIEF DESCRIPTION OF THE INVENTION

The invention is defined by the independent claim 1. As introduced in the preceding section, a first object of the present invention relates to a device for selectively eradicating or slowing down the progression of tumors based on the delivery of pulsed AC electric fields to a region of a patient's body comprising one or more tumors. This device comprises at least two electrodes and a generator that supplies AC electric field pulses or bursts which induce extracellular calcium uptake by living cells, being more harmful to tumor cells than to healthy cells, hampering the tumor progression and/or eradicating it.

As stated, the AC electric field is pulsed (i.e., applied in the form of bursts) rather than continuous, which allows for the delivery of relatively large magnitude fields through the human body in a safe manner. These AC electric pulses have moderate amplitudes which are sufficient to induce substantial calcium uptake but not as high as to cause damage by electroporation or unsought electrostimulation.

Albeit mildly, cell permeability does increase for ions and small molecules when AC fields of moderate magnitude are applied during long exposures. Remarkably, under these circumstances, and although these waveforms are much less effective than DC pulses of high magnitude for standard electroporation, a very substantial uptake of extracellular calcium, which can cause several and diverse effects in favor of the eradication of tumors, takes place. It is to be noted that local concentrations of intracellular calcium ($Ca_2^+$ ions) are considered to be ubiquitous intracellular signals responsible for controlling numerous cellular processes and that calcium is much more abundant extracellularly than intracellularly.

In view of the above features, the device preferably comprises a plurality of electrodes and an AC generator electrically connected thereto, wherein the electrodes are adapted to encompass a region of a subject's body comprising one or more tumors, and the AC generator is adapted to continuously supply pulsed AC electric fields during a period of at least two hours, wherein said AC electric fields comprise:

- a fundamental frequency between 10 kHz and 1 MHz;
- an amplitude between 10 V/cm and 500 V/cm;
- duty cycles between 0.001% and 10%.

Advantageously, the device achieves the delivery of sustained pulsed alternating fields that cause a mild perturbation in the cellular membrane, thereby steadily perturbating cellular homeostasis hampering tumor progression, until eradicating them. Besides, the pulses can be delivered non-locally, i.e., through a region of a subject's body in which more than one tumor may be comprised. As detailed above, these pulsed alternating fields applied during at least two hours (in order to ensure their application during the most sensitive phases of the tumor cell life cycle) induce extracellular calcium uptake, which is more harmful to tumor cells or to rapidly dividing cells than to healthy cells. Other therapeutically beneficial effects of these sustained pulsed alternating fields are an increment in cellular membrane permeability to chemotherapeutic agents, and a disruption of the endothelial lining of tumor vasculature that hinders angiogenesis, causes ischemia, or enhances the transport of chemotherapeutic agents from the vessels to the tumor parenchyma.

The fundamental frequency of the AC electric field is, preferably, larger than 10 kHz to prevent electrochemical reactions at the electrodes and to prevent unsought electrostimulation and lower than 1 MHz to ensure that it induces a sufficient transmembrane voltage magnitude for perturbating the cellular membrane. Another additional advantage of relatively high frequencies, compared to DC or low frequencies, is that the electrode-tissue impedance drops at high frequencies thus facilitating electrical contact and diminishing the power requirements of the generator. It is to be noted that, due to the passive electrical properties of the membrane and of the extracellular and intracellular media, the transmembrane voltage exhibits a low-pass filter behavior in relation to the applied electric field.

The amplitude of the AC electric field is larger than 10 V/cm, so as to substantially perturbate the cellular membrane, yet lower than 500 V/cm, to prevent damaging healthy cells by irreversible electroporation and to avoid unsought electrostimulation. Lower field magnitudes also perturbate the cellular membrane but their impact on cellular homeostasis is too low and it is counteracted by regulatory or repair mechanisms of the cells. Lower field magnitudes also induce calcium uptake but at a low rate that is counteracted by the several mechanisms that living cells possess to control the calcium concentration levels within the cytoplasm and various organelles.

The AC electric fields (with fundamental frequency $f$ and with amplitude $A$) generated by the device of the invention are advantageously pulsed, i.e., they consist of bursts delivered with duration $B$, and repetition frequency $F$. The product $\boldsymbol{F} \times \boldsymbol{B}$ is the duty cycle ($D$). The duty cycle of the pulsed AC electric field can be adjusted to prevent excessive heating of living tissues by resistive heating. In particular, the following known expression can be used to such purpose in the case of sinusoidal waveforms:

$$\mathrm{SAR} = \frac{\sigma A^2}{2\rho} FB,$$

where SAR is the dissipated heat per unit of mass (W/kg), $\sigma$ is the conductivity of the living tissue at frequency $f$ and $\rho$ is the mass density of the living tissue. Electrical safety standards dictate maximum SAR local levels between 2 W/kg and 20 W/kg. However, limitations dictated by electrical safety standards can be exceeded for medical treatments. Assuming a maximum local SAR of 100 W/kg (which would cause a temperature increase of a few Celsius degrees in most soft living tissues), a typical tissue density of 1000 kg/m$^3$, and a typical soft tissue conductivity ranging from 0.1 S/m to 1.5 S/m, this indicates that, for the maximum field amplitude of 500 V/cm, the maximum duty cycle would be 0.005 %. On the other hand, assuming the lightest treatment with a maximum SAR of 2 W/kg and the minimum field amplitude of 10 V/cm, the maximum duty cycle would be 4%. Taking into account uncertainties and the fact that the SAR limitation is not only relevant at the site of treatment but also at others where the electric field can be larger (e.g., close to the electrodes edges), the range of possible duty cycles is expanded to the range from 0.001% to 10% in a safe manner.

In general, cancer cells can have cell cycle durations that are much shorter than the typical cell cycle durations of healthy cells. However, even in cancer cells, the cell cycle lasts at least a few hours. As mentioned above, exposures longer than two hours to maximize the probability of the induced perturbation occurring in the cell phase or phases during which the cancer cells are more susceptible to damage. Furthermore, in the case of combined use of the system with other treatments (e.g., chemotherapy, or immunotherapy), exposures longer than two hours also ensure that the induced perturbation occurs while these other therapies are effective.

In a preferred embodiment of the invention, the AC electric field pulses are shorter than 200 ms, in order to minimize unsought stimulation and to be able to apply larger electric field magnitudes for higher treatment efficacy. This threshold corresponds to the integration time indicated by the IEEE safety standard for computing the maximum exposure to avoid unsought stimulation.

In a further preferred embodiment of the invention, the generator is programmed to supply the pulsed AC electric fields during two or more active phases, each of said active phases being separated from the previous and next active phase by a period of time corresponding to a rest phase in which no AC electric fields are supplied. More preferably, each of the active phases is longer than 2 hours and shorter than 24 hours, and each of the rest phases is longer than 2 hours and shorter than 7 days. This very low pace intermittence maximizes the chances of damaging and killing cancer cells during their cell cycles while allowing healthy cells to recover. The durations of the active and resting phases do not necessarily have to be constant. Similarly, the amplitude of the electric field during the active phases does not need to be constant.

In a further preferred embodiment of the invention, the AC electric fields comprise a sinusoidal waveform or a waveform without a DC component (such as symmetric or asymmetric biphasic square pulses, saw pulses, triangular pulses, etc.) to prevent electrochemical reactions at the electrodes.

In a further preferred embodiment of the invention, the generator comprises a blocking capacitor connected in series with the electrodes, said blocking capacitor being adapted to prevent passage of a DC current and to enable the flow of an AC electric current associated with the AC electric fields.

In a further preferred embodiment of the invention, the device further comprises amplitude modulation means such that the amplitude of the AC electric fields can be modulated. This allows the pulses to comprise a soft start and/or a soft end such that harmonics and thus electromagnetic compatibility issues and unsought electrostimulation are minimized.

In a further preferred embodiment of the invention, the electrodes are dry electrodes and comprise protrusions and/or indentations in order to facilitate the electrical coupling with the living tissue.

In a further preferred embodiment of the invention, the electrodes are short-circuited or arranged in pairs such that the AC electric fields deployed through said arrangements can be switched sequentially. This allows for covering a larger body region, for intensifying the treatment in a sub-region, and/or for exposing the target tissues to different field orientations.

In a further preferred embodiment of the invention, the generator includes a monitoring circuit adapted to measure the voltage and current applied to detect whether the electrodes are in contact with a subject's body. The monitoring circuit may be connected to one or more temperature sensors comprised by the electrodes, in order to detect the temperature distribution therein. For instance, this may be carried out by means of a matrix of temperature sensors (such as thermistors among others) attached to, or embedded into, the electrodes. This enables detecting thermal anomalies caused, among other reasons, by a partial detachment of the electrodes.

In a further preferred embodiment of the invention, the generator and the electrodes are embedded in a wearable piece of garment such as a headband, cap, bandana, t-shirt, jersey, underwear, bib shorts, sleeves, etc., depending on the location of the tumor/s, and/or are implemented, for instance, with metals well tolerated by the skin (e.g. stainless steel) or, for greater comfort and conformability, with conductive fabrics. The generator may be carried by the subject shaped as, or being part of, a backpack or a pocket belt, among other options. This embodiment allows to apply the treatment for days, weeks or months, while subjects carry out their daily activities.

In a further preferred embodiment of the invention, the generator and/or the electrodes are adapted to be implanted in a living body for cosmetic and comfort of the subject. Preferably, for minimal invasiveness, all the elements comprised by the device may be subcutaneously implanted.

In a further preferred embodiment of the invention, the device further comprises delivery means adapted to deliver a substance into the region encompassed by the electrodes during the suppliance of the AC electric fields. This allows for using the device in combination with other treatments such as chemotherapy, radiotherapy, or immunotherapy for maximizing antitumor efficacy.

A second object of the present disclosure relates to a non-claimed method for eradicating or slowing down the progression of tumors based on the delivery of pulsed AC electric fields to a region of a patient's body comprising one or more tumors, which preferably comprises the operation of a device according to any of the embodiments described in this document.

In a preferred embodiment of the disclosure, the non-claimed method comprises the realization of the following steps:

- deploying the electrodes in a region of a subject's body, the region comprising one or more tumors;
- connecting the electrodes to the AC generator; and
- continuously supplying pulsed AC electric fields from the generator to the electrodes during a period of at least two hours, wherein said AC electric fields comprise:
- a fundamental frequency between 10 kHz and 1 MHz;
- an amplitude between 10 V/cm and 500 V/cm;
- duty cycles between 0.001% and 10%.

The electrodes can be deployed either in an external or in an internal region of the subject's body. In the former case, the electrodes are surface electrodes whereas in the latter case the electrodes (and, optionally, the AC generator) are adapted to be implanted inside the subject's body as described in some of the embodiments above.

In a further preferred embodiment of the disclosure, within the disclosed method, the pulsed AC electric fields are applied in treatment sessions.

In a further preferred embodiment of the disclosure the pulsed AC electric fields are applied continuously during days, weeks or months.

In a further preferred embodiment of the disclosure the pulsed AC electric fields are applied in combination with chemotherapy, radiotherapy, and/or immunotherapy.

In the context of the present invention the terms "pulse" and "burst" are used equivalently.

Also, in the context of the present invention, "the electrodes being adapted to encompass a body region" refers primarily to the arrangement of the electrodes in which said body region is encompassed by the electric field/s generated between the electrodes.

Finally, in the context of the present invention, a distinction is made between "continuous application of an AC electric field" and "continuously delivered pulsed AC electric fields". The former refers to the application of an AC electric field which is not in the form of bursts or pulses, while the latter refers to continuously supplied pulsed AC electric fields. This means that, in the latter case, although an AC electric field is supplied continuously (especially in cases where the amplitude of the AC electric field does not reach 0 between bursts) it is in the form of pulses or bursts, such that the advantages described above are obtained.

## BRIEF DESCRIPTION OF THE DRAWINGS

The above-detailed and other features and advantages of the present invention will become apparent in view of the following non-limiting description of different embodiments of the invention, made with reference to the accompanying drawings, where:

Figure 1 shows a schematic representation of the device according to an embodiment of the invention in the context of a preferred application of said device for eradicating and/or for slowing down the progression of tumors.

Figure 2 illustrates the waveform of the pulsed AC electric fields delivered by a device according to an embodiment of the invention, along with some of the basic parameters describing the same: duration of the pulse, repetition frequency, fundamental frequency and amplitude.

Figure 3 shows the intracellular calcium concentration during the delivery of pulsed AC fields by means of a device according to an embodiment of the invention (left) and during the delivery of moderate alternating fields applied continuously according to some of the methods in the art (right).

Figure 4 illustrates the application pattern of the pulsed AC electric fields delivered by a device according to an embodiment of the invention in which the generator is configured to supply said fields during several active phases, each of said active phases being separated from the previous and next active phase by a period of time corresponding to a rest phase in which no AC electric fields are supplied.

Figure 5 illustrates the pulsed AC electric fields delivered by a device according to an embodiment of the invention in which the amplitude of said fields within a pulse is modulated so that a soft start and a soft end are delivered.

Figure 6 illustrates a device according to an embodiment of the invention applied to the treatment of brain tumors in which the electrodes are arranged in pairs such that the AC electric fields deployed through said arrangements can be switched sequentially for intensifying the treatment in a region.

Figure 8 shows a device according to an embodiment of the invention in which an array of temperature sensors is embedded in the electrodes.

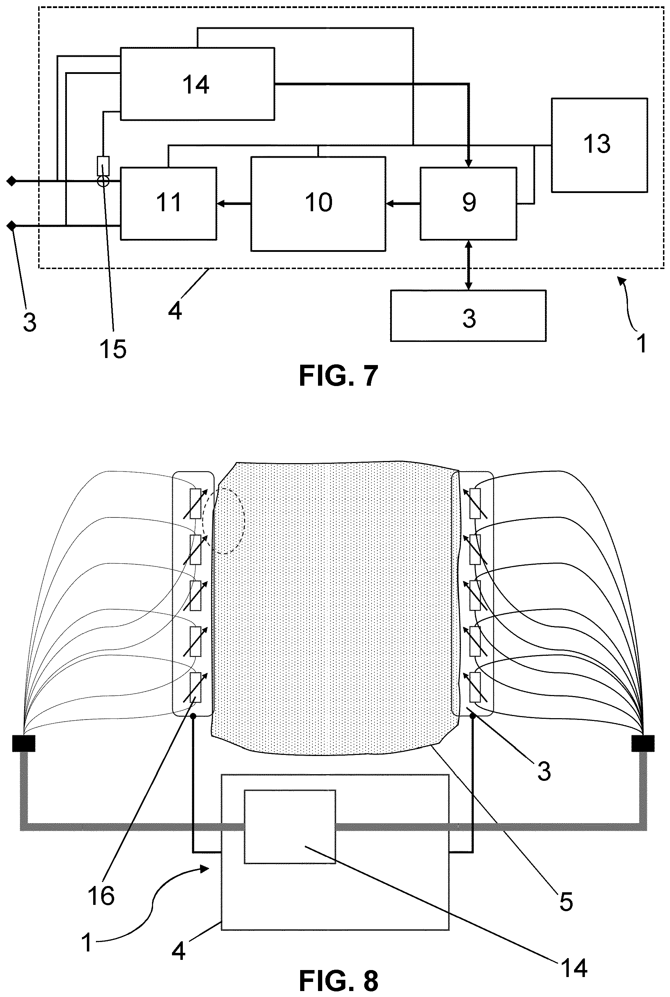

Figure 7 shows a schematic representation of an architecture for the generator comprised in a device according to an embodiment of the invention.

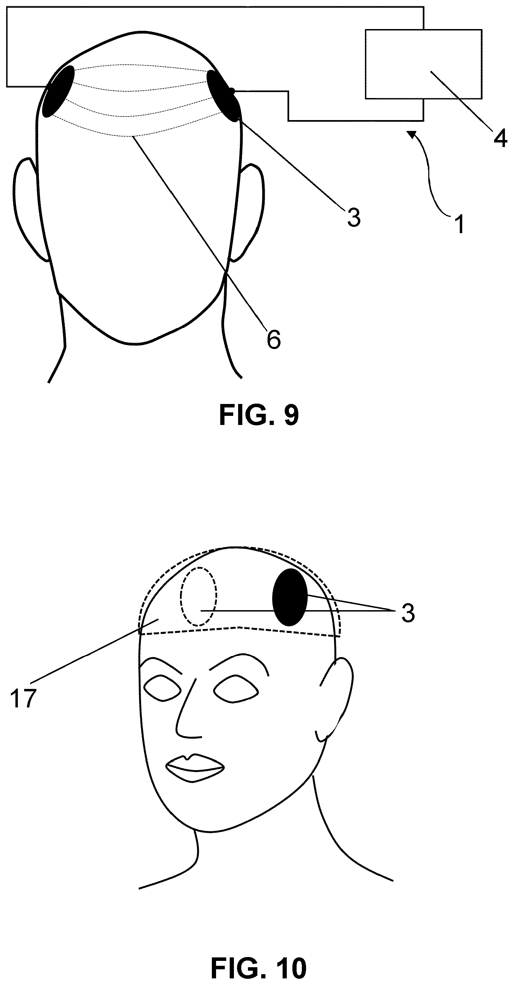

Figure 9 shows a device according to an embodiment of the invention applied to the treatment of brain tumors by placing the electrodes at opposite sides of a subject's head.

Figure 10 shows a device according to an embodiment of the invention applied to the treatment of brain tumors in which the electrodes are comprised in an externally passive fixation system such as a cap.

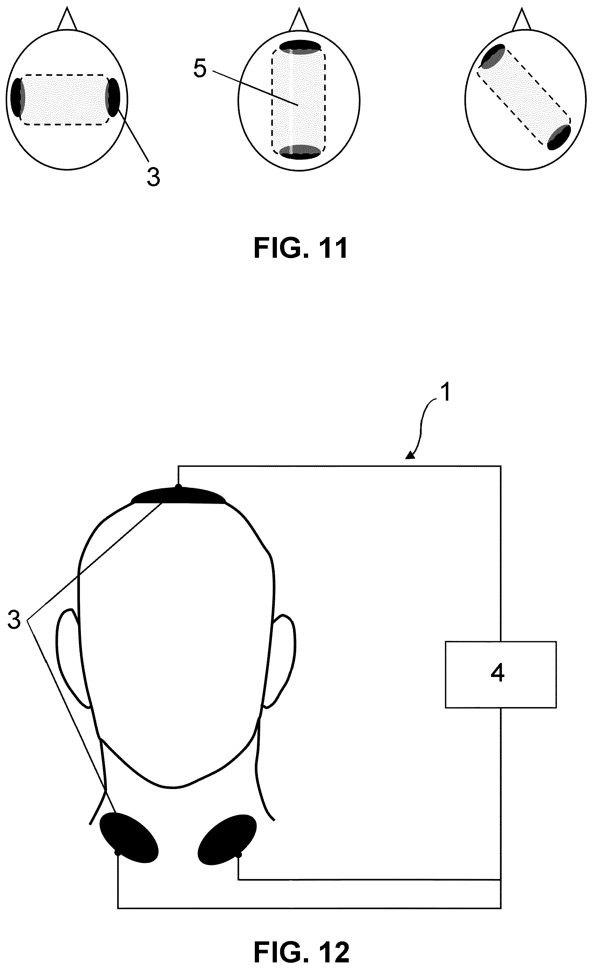

Figure 11 illustrates different possible placements for the electrodes of a device according to an embodiment of the invention applied to the treatment of brain tumors.

Figure 12 shows a device according to an embodiment of the invention applied to the treatment of brain tumors by placing one electrode at the top of a subject's head and two electrodes on the next of said subject.

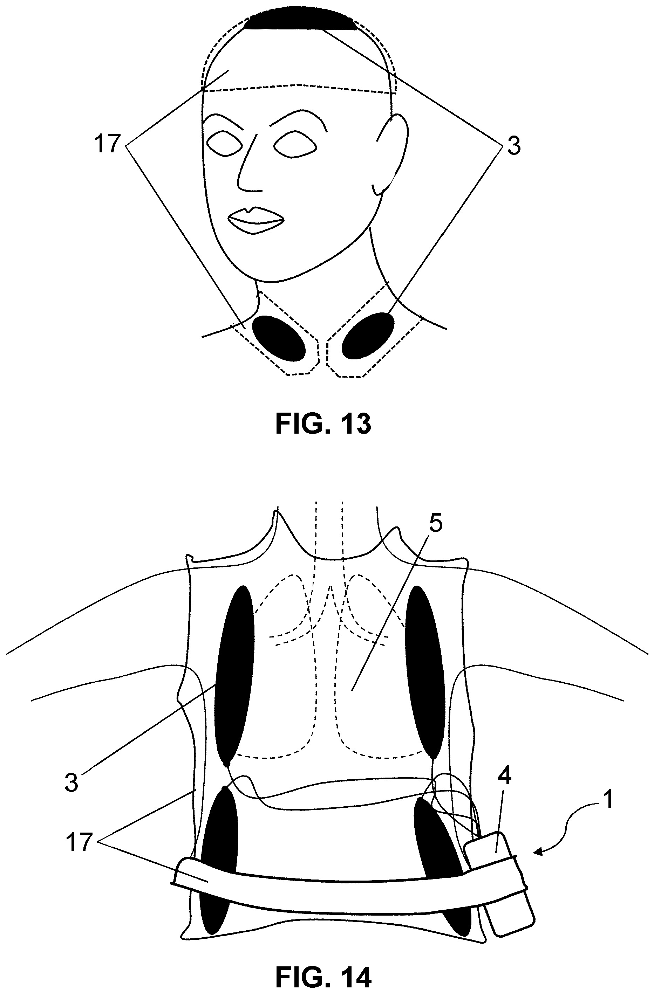

Figure 13 shows the same device as Figure 12 in which the electrodes are comprised in externally passive fixation systems.

Figure 14 shows a device according to an embodiment of the invention applied to the treatment of tumors present in the thorax or abdomen in which said device is embedded in a piece of garment adapted to be worn by a subject.

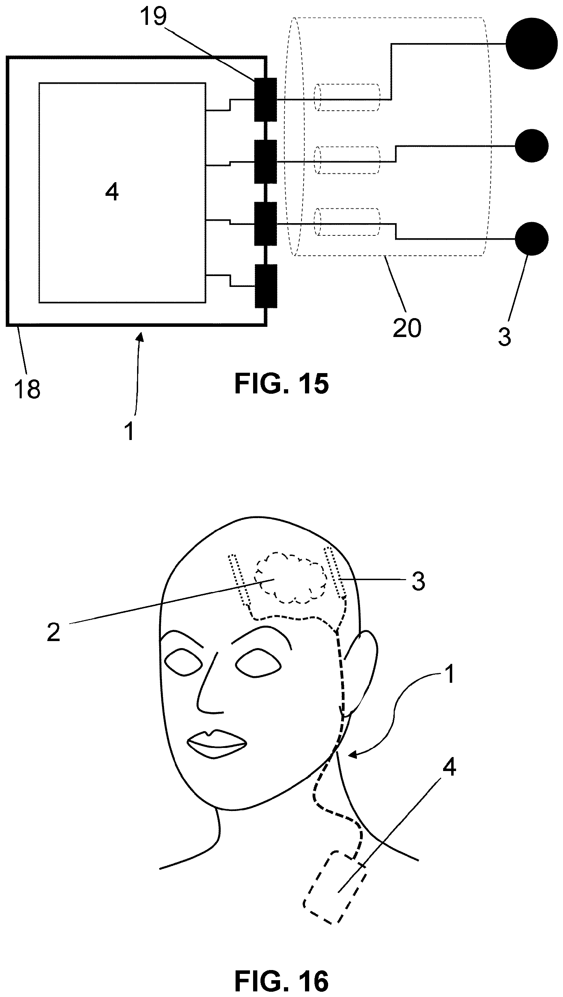

Figure 15 shows a device according to an embodiment of the invention in which the device is adapted to be implanted into a subject's body.

Figure 16 shows a device according to an embodiment of the invention subcutaneously implanted into a subject's head and neck.

## NUMERIC REFERENCES USED IN THE DRAWINGS

| 1 | Device |
| --- | --- |
| 2 | Tumor |
| 3 | Electrodes |
| 4 | Generator |
| 5 | Body region (comprising one or more tumors) |
| 6 | Electric field lines |
| 7 | Burts/pulse |
| 8 | Switching elements |
| 9 | Digital Control Unit (DCU) |
| 10 | Digital waveform generator |
| 11 | Voltage amplifier |
| 12 | Interface or communications stage |
| 13 | Power supply |
| 14 | Monitoring subcircuit |
| 15 | Electric sensor |
| 16 | Temperature sensor |
| 17 | Wearable piece of garment |
| 18 | Hermetic capsule |
| 19 | Hermetic electric connections |
| 20 | Insulation capsule |

## DETAILED DESCRIPTION OF THE INVENTION

Different embodiments for the system of the invention will be now described for illustrative, but not limiting, purposes.

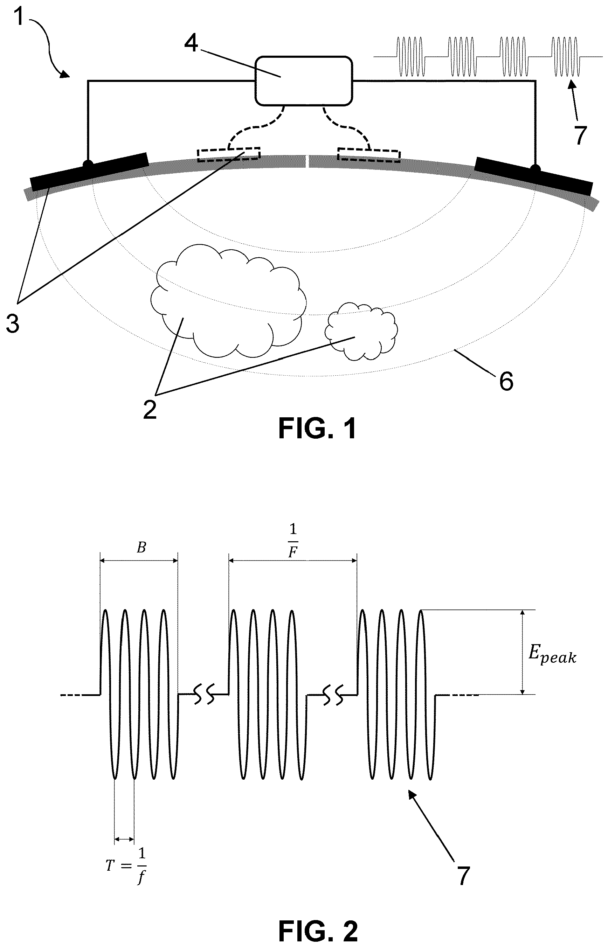

The main elements comprised in a device (1) for eradicating or slowing down the growth of tumors (2) according to an embodiment of the invention are shown in Figure 1, along with a representation of a preferred application of said device for the treatment of tumors (2). The device (1) comprises a plurality of electrodes (3) and an AC generator (4) electrically connected to said electrodes (3), by using, for example, standard wiring, although other techniques known in the art may also be used.

The plurality of electrodes (3) may consist of at least two electrodes (3) placed in such a way or adapted to encompass a body region (5) in which one or more tumors (2) are comprised. As seen in Figure 1, this is to be understood as meaning that the electric field generated between the electrodes (represented in the Figure by electric field lines (6)) is located in a region of the subject's body (5) in which the tumor/s (2) are comprised. Also, as seen in this Figure, more electrodes (2) or sets of electrodes (2) may be comprised by the device in order to tailor the corresponding treatment depending on the specificities of each application.

Figure 1 also presents a schematic view of the electric field that is supplied by the AC generator (4) to the electrodes (3) (and thus by the electrodes (3) to the corresponding body region (5)). This field is a pulsed AC electric field, i.e., an alternating current electric field applied in the form of bursts (7) as shown in more detail in Figure 2.

The fundamental frequency of the AC electric field, $f = \dfrac{1}{T}$, with $T$ being the period of the oscillating field, is comprised between 10 kHz and 1 MHz. This range of frequencies is selected such that neither electrochemical reactions at the electrodes (3) nor unsought electrostimulation takes place but also such that sufficient transmembrane voltage magnitude for perturbating the cellular membrane is applied. Furthermore, at these frequencies the electrode-tissue impedance drops facilitating electrical contact and diminishing the power requirements of the AC generator (4). On the other hand, the amplitude (i.e., peak amplitude) of the AC electric fields is comprised between 10 V/cm and 500 V/cm to substantially perturbate the cellular membrane without damaging healthy cells by irreversible electroporation. Lower field magnitudes also perturbate the cellular membrane but their impact on cellular homeostasis is too low and it is counteracted by regulatory or repair mechanisms of the cells.

Regarding the bursts (7), they are characterized by two fundamental parameters: duration ($B$), which, in the present embodiment, is preferred to be shorter than 200 ms, and frequency of repetition ($F$). The product $F \times B$ corresponds to the so-called duty cycle ($D$). The duty cycle of the pulsed AC electric fields is adjusted to prevent excessive heating of living tissues by resistive heating. In particular, the range of possible duty cycles employed in the device of the invention according to this embodiment is comprised between 0.001% and 10%, in order to ensure the safety of healthy tissues.

Although not shown in Figure 2, in an embodiment of the invention, the amplitude of the pulsed AC field may not drop to zero in between the bursts (7). It may be maintained, instead, at a low constant level lower 10 V/cm in order to maximize treatment efficacy by combining the effects of pulsed AC fields with TTFields.

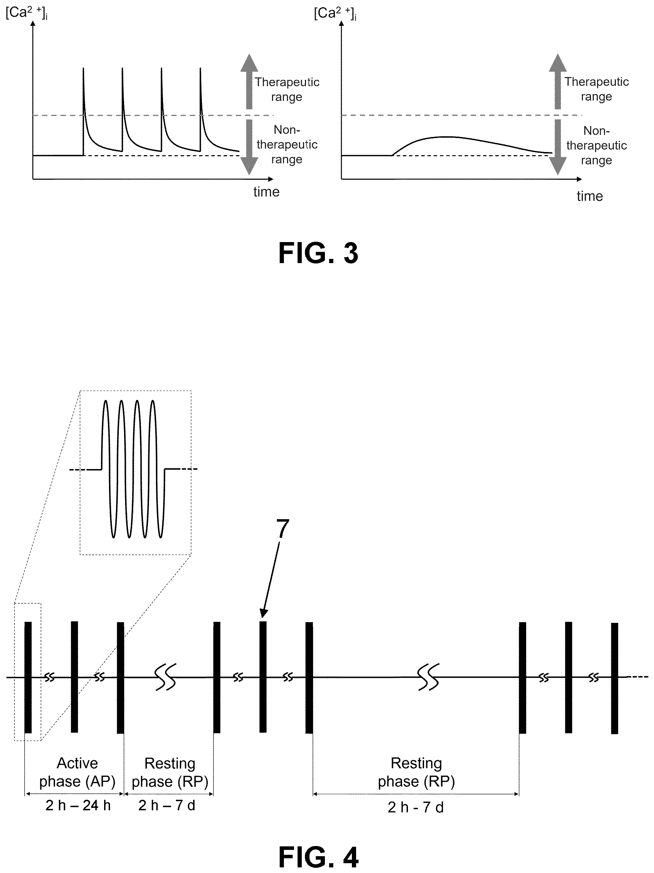

Figure 3 illustrates the main advantages of using a device (1) of these characteristics in the treatment of tumors (2). In the left panel of said Figure, the intracellular calcium concentration during the delivery of pulsed AC electric fields by means of a device (1) according to the present invention is shown. As seen in said Figure, the intense bursts (7) cause transient perturbations in the cell membrane's permeability that cannot be counteracted immediately by the cells. In particular, the intense bursts (7) cause a substantial uptake of calcium that is not immediately counteracted by the homeostasis mechanisms of the cell thus reaching therapeutic intracellular calcium concentration levels, i.e., killing or slowing down the growth the cancerous cells.

The right panel shows, in turn, the intracellular calcium concentration during the delivery of moderate-intensity (i.e., moderate-amplitude) AC fields applied in a continuous manner (not in bursts (7)) as is the case in techniques known in the art. The calcium concentration levels never reach, in this case, the therapeutic range, thus being not effective in the treatment of tumors (2). Therefore, the use of a device (1) according to the present invention increases relevantly the efficiency of the treatment of tumors (2) due to the combination of characteristics detailed in the previous paragraphs regarding the pulsed AC electric fields. This allows for calcium uptake by the cancerous cells to be sufficient to induce the death of said cells or, at least, to stop or slow down their growth and replication with minimum invasiveness for the healthy tissues.

This effectiveness is increased in an ever more relevant manner when the application of the AC electric fields lasts at least for two hours so that all the phases of the life cycle of the cancer cells (including the most sensitive ones to damage) are comprised within the treatment. In order to allow this, the AC generator (4) in the device (1) of the invention is adapted to supply continuously the pulsed AC electric fields during a period of at least two hours.

Furthermore, in an embodiment of the invention, the AC generator (4) is further configured to supply the pulsed AC electric fields during two or more active phases, each of said active phases being separated from the previous and next active phase by a period of time corresponding to a rest phase in which no AC electric fields are supplied. This time-pattern can be seen in Figure 4, where each of the active phases is longer than 2 hours and shorter than 24 hours, and where each of the rest phases is longer than 2 hours and shorter than 7 days. The total treatment may last, therefore, hours, days, or weeks, depending on the specific application, and can be applied in treatment sessions lasting between minutes and hours that can be periodic (with periods of hours, days, weeks or months) or non-periodic. Also depending on the specificities of the application, the durations of the active and resting phases do not necessarily have to be constant. Similarly, the amplitude of the electric field during the active phases does not necessarily need to be constant. After an initial period in which intense treatment regime is applied with the intention of killing tumor cells, it may be beneficial to apply a more moderate treatment regime (e.g., lower magnitudes, shorter active phases and longer resting phases) over an extended period to prevent cancer recurrence or slow its progression. As mentioned, this adaptable intermittence maximizes the chances of damaging and killing cancer cells during their cell cycles while allowing healthy cells (with longer life cycles) to recover.

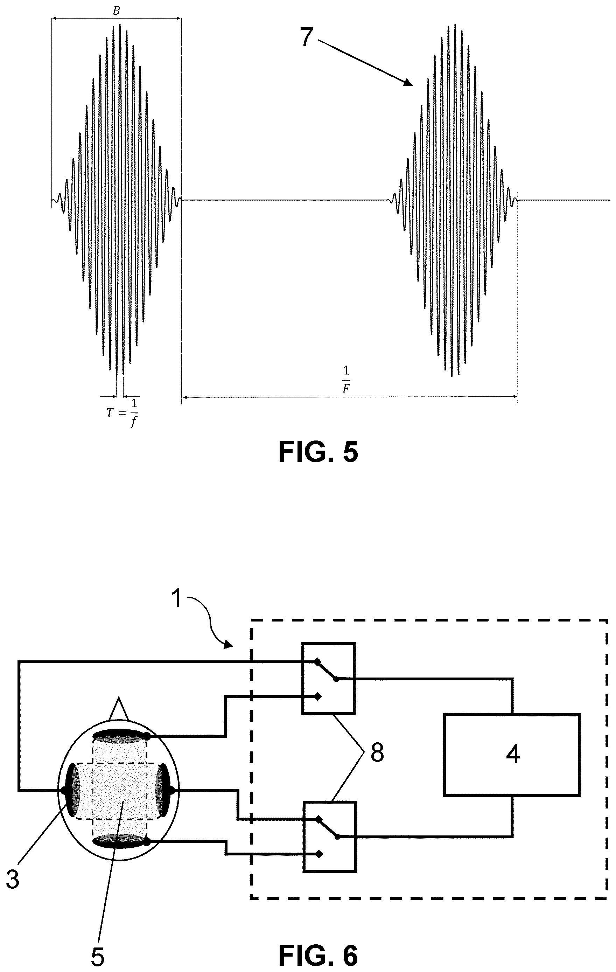

In terms of adaptability, the AC generator (4) may comprise, according to an embodiment of the invention, modulating or windowing means to regulate the amplitude of the AC electric fields within each pulse. These modulating means may be any standard modulating means known in the art. The modulation of the amplitude of the fields allows for a soft start and/or end of each pulse as seen in Figure 5. In said Figure, the effect of a Tukey window/modulation is represented, also known as the cosine-tapered window. Other known spectrally advantageous windows that may be applied in the present invention include the Hann window, the Hamming window, the Blackman window, the triangular window, the Gaussian window and the Kaiser window.

Figures 2, 4 and 5 show pulsed AC fields comprising a sinusoidal waveform. Nevertheless, other possible waveforms, as long as they comprise no DC component, may be also used without departing from the scope of the invention.

Figure 6 shows an embodiment of the device (1) of the invention in which the electrodes (3) are arranged in pairs and are short-circuited by means of switching elements (8) inserted in the connections between the AC generator (4) and said pairs of electrodes (3). The switching elements (8) may consist in electromechanical relays or solid-state devices such as transistors and may be governed by a Digital Control Unit (DCU) to sequentially connect the output of the AC generator (4) to the pairs of electrodes (3) (or to pairs of sets of electrodes (3) in which each set is formed by short-circuiting the electrodes (3) therein). Switching is produced in between bursts (7) and preferably, but not necessarily, after each burst (7). The amplitude of the current and/or the voltage applied in each electrode combination can be adjusted for producing the same electric field.

Specifically, Figure 6 shows two pairs of electrodes (3) arranged perpendicular to each other so that the electric fields generated across each pair of electrodes (3) overlap in a central region. In this embodiment, the AC electric fields are deployed through said pairs and are switched sequentially to expose the target tissues (the mentioned central region) to different field orientations. Other possible applications of such a configuration include covering larger body regions (5), and/or intensify the treatment in a specific sub-region.

Regarding the AC generator (4), it may comprise several possible architectures within the scope of the present invention. It is known that electric fields are induced in living tissues when either a voltage-controlled waveform or a current-controlled waveform are delivered across the electrodes. It is also known how to compute the electric field that results from the delivery of a voltage or a current. Coarsely, the electric field magnitude will correspond to the ratio between the applied voltage and the distance between the electrodes and the field direction will be aligned with the axis between both electrodes. For accurate estimations, known numerical methods can be applied to compute the electric field distribution in tissues.

In a preferred embodiment of the invention, the AC generator (4) comprises of a Digital Control Unit (DCU) (9) such as a computer processing unit (CPU), a field-programmable gate array (FPGA) or any equivalent general digital microprocessor equipped with processing and timing means, adapted to autonomously govern the operation of a digital waveform generator (10) whose output is amplified by a voltage amplifier (11). The DCU (9) may also be connected to an interface or communication stage (12) in order to allow for the exchange of information with a user of the device (1) and/or to allow for manual control of the device (1) by a user. It may also be adapted to be programed with different treatment patterns (fundamental frequencies, amplitudes, duty cycles and duration of the active and rest phases) so that the whole treatment is delivered autonomously (i.e., without intervention of a user). All the circuitry is powered by a power supply (13) which, in turn, preferably comprises a battery or other supplying means.

Optionally the generator further comprises a monitoring subcircuit (14) comprising, in turn, an electric sensor (!5) connected to the output of the voltage amplifier (11) which is able to sense the applied voltage and current in order to detect whether the electrodes (3) are properly contacted with the living tissues. By determining the applied voltage and current, the monitoring subcircuit (24) can compute the impedance across the electrodes (3) and, if the calculated value is out of a preestablished range, warn the user to indicate that the connection is not adequate. The presented features regarding possible architectures of the AC generator (4) (except for the switching elements (8)) are schematically depicted in Figure 7.

Another embodiment of the present invention includes at least one temperature sensor (16) comprised in and/or at the electrodes (3) and connected to the monitoring subcircuit (14) comprised by the AC generator (4). For example, Figure 8 shows an embodiment of the invention in which an array (or matrix) of temperature sensors (16), such as thermistors or others known in the art, are embedded within and/or mounted on the electrodes (3) to detect thermal anomalies caused, for instance, by a partial detachment of the electrodes (3).

All the features and embodiments detailed in the above paragraphs may be implemented in different configurations. More specifically, the electrodes (3) and the AC generator (4) can be arranged in different positions with respect to each other and with respect to the subject's body region (5) of interest, precisely depending on the specific application of the device (1).

For instance, Figures 9-13 show different embodiments of the device (1) adapted for the treatment of brain tumors (2). Figure 9 illustrates the most basic implementation of the device (1) for such purpose by placing the electrodes (3) at opposite sides of the head of a subject, allowing the pulsed AC electric fields to be applied across the region (5) in which the tumor or tumors (2) are present. This same implementation may be carried out by embedding or attaching the electrodes (3) to a wearable piece of garment (17) such as a cap, among other options, as seen in Figure 10. In all cases, the electrodes (3) can be placed at different positions (see Figure 11 for different electrodes placements) for treating different regions (5), in this case, of the brain, and can remain fixed during treatment or relocated if necessary.

Another possible configuration for the treatment of brain tumors (2) corresponds to that shown in Figures 12-13. There, three electrodes (3), connected to the AC generator (4), are used and placed over the subject's body. Specifically, one of said electrodes (3) is placed at the top of the subject's head, whereas the other two are placed at both sides of the subject's neck. Two electrodes (3) placed are placed at the neck for symmetry reasons and because it may be advantageous to use two electrodes (3) for minimizing the contact impedance and for monitoring such contact impedance, although the invention would still be operative with a single electrode (3). This arrangement for treating brain tumors (2) is cosmetically advantageous: the top electrode (3) can be hidden beneath a wig, or a scarf and the bottom electrodes (3) (neck) can be hidden behind the collar of a shirt.

This later case is shown in Fig. 13, in which, similarly as in Figure 12, the electrodes (3) are embedded or attached to wearable pieces of garment (17). More specifically, the electrodes (3) can be part of an electrically passive structure (such as a headset-like structure, headband, helmet, cap, bandana or scarf) that facilitates mechanical fixation as well as donning and doffing and that also serves for cosmetic purposes. Examples of electrically passive structures are well known in the art, for instance, for electroencephalography (EEG) and transcranial-direct current stimulation (tDCS).

Furthermore, the electrodes (3) can be implemented in different ways. In a preferred embodiment, the electrodes (3) are of the type known as "dry electrodes (3)", meaning that they do not require any fluid of gel to improve their electrical contact with the skin. The use of high frequencies for power transfer and communications facilitates the use of such dry electrodes (3) because the electrical conduction across interface between the electrode (3) and the tissues is predominantly capacitive and not galvanic. In fact, it is possible to employ electrodes (3) with a thin dielectric layer on its surface. These electrodes (3) can be implemented, for instance, with metals well tolerated by the skin (e.g., stainless steel) or, for greater comfort and conformability, with conductive fabrics or with conductive polymers (e.g carbon filled rubber). In order to facilitate electrical contact across hair, the electrodes (3) can contain protrusions in the shape of bristles or blunt spikes.

All the above-mentioned features regarding embodiments of the device (1) of the invention tailored to their application in the treatment of brain tumors (2) may be directly translated into the treatment of tumors (2) in other regions (5) of a subject's body. For instance, and as a non-limiting example, Figure 14 shows an embodiment of the present invention in which electrodes (3) are placed at opposite sides of a subject's thorax in which the tumor or tumors (2) are comprised. The electrodes (3) are embedded into a wearable piece or garment (17) (a vest) that is worn by the subject, and the (battery-powered) AC generator (4) is mounted on a belt that may also be worn by the subject, for comfort.

This configurations and dispositions can be applied to any part of the body of a subject (brain, thorax, abdomen, limbs, etc.) in which tumors are comprised.

Finally, for cosmetic and comfort reasons in the case of very long treatments, instead of embedding or attaching the electrodes (3) to a wearable piece of garment (17), they can be implanted into de subject's body, either alone or together with the AC generator (4). In the embodiments in which the whole device (1) is implanted, the AC generator (4) is enclosed in a hermetic capsule (18) electrically connected by means of hermetic electrical connections (19) and through feedthroughs to wires comprised in an insulation capsule (20) connected to the electrodes (3) (see Figure 15). The generator can be powered by a battery contained within the same hermetic capsule (18) or it can be powered externally by known methods for wireless power transfer such as inductive coupling or ultrasonic coupling.

Preferably, for minimal invasiveness, all these elements (the AC generator (4), the wires, and the electrodes (3)) are subcutaneously implanted as depicted in Figure 16 for the case of a brain tumor (2) treatment, and similarly applied when placed in other regions (5) of the body.

A device according to any of the above-described embodiments may be operated as one or more steps of a non-claimed method for eradicating or slowing down the progression of tumors based on the delivery of pulsed AC electric fields to a region of a patient's body comprising one or more tumors.

This non-claimed method comprises deploying the electrodes in a region of a subject's body comprising one or more tumors, connecting the electrodes to the AC generator and continuously supplying pulsed AC electric fields from the generator to the electrodes during a period of at least two hours, wherein said AC electric fields comprise the above-mentioned characteristics: a fundamental frequency between 10 kHz and 1 MHz, an amplitude between 10 V/cm and 500 V/cm and duty cycles between 0.001% and 10%.

The electrodes can be deployed either in an external or in an internal region of the subject's body according to corresponding embodiments of the device of the invention. In the former case, the electrodes are surface electrodes whereas in the latter case the electrodes (and, optionally, the AC generator) are adapted to be implanted inside the subject's body as described in some of the embodiments above.

In a further preferred embodiment of the disclosure, within the disclosed method, the pulsed AC electric fields are applied in treatment sessions, and/or continuously during days, weeks or months, and/or they may be applied in combination with chemotherapy, radiotherapy, and/or immunotherapy, or other techniques known in the art in order to enhance the effectiveness of the treatment.

## Claims

1. Device (1) for selectively eradicating and/or for slowing down the progression of tumors (2) comprising:

   - a plurality of electrodes (3); and
   - an AC generator (4) electrically connected to the electrodes (3);
   wherein the electrodes (3) are adapted to encompass a region (5) of a subject's body comprising one or more tumors (2);
   wherein the AC generator (4) is adapted to supply a plurality of AC electric field pulses (7) comprising:

      - a fundamental frequency between 10 kHz and 1 MHz;
      - an amplitude between 10 V/cm and 500 V/cm; and
      - duty cycles between 0.001% and 10%;

   and wherein the AC generator (4) is further adapted to supply the AC electric field pulses (7) continuously during a period of at least two hours.

2. Device (1) according to the preceding claim, wherein the duration of the AC electric field pulses (7) is substantially equal to or shorter than 200 ms.

3. Device (1) according to any of the preceding claims, wherein the AC generator (4) is configured to supply the AC electric field pulses (7) during two or more active phases, each of said active phases being separated from the previous and next active phase by a period of time corresponding to a rest phase in which no AC electric field pulses (7) are supplied.

4. Device (1) according to the preceding claim, wherein each of the active phases is longer than 2 hours and shorter than 24 hours, and wherein each of the rest phases is longer than 2 hours and shorter than 7 days.

5. Device (1) according to any of the preceding claims, wherein the AC electric field pulses comprise a sinusoidal waveform or a waveform without a DC component.

6. Device (1) according to any of the preceding claims, wherein the generator is adapted to maintain a constant amplitude smaller than 10 V/cm of the pulsed AC fields in between the bursts (7).

7. Device (1) according to any of the preceding claims, wherein the AC generator (4) comprises a blocking capacitor connected in series with the electrodes (3), said blocking capacitor being adapted to prevent passage of a DC current and to enable the flow of an AC electric current associated with the AC electric fields.

8. Device (1) according to any of the preceding claims, further comprising amplitude modulation means such that the amplitude of the AC electric fields can be modulated in order to obtain a soft start and/or a soft end.

9. Device (1) according to any of the preceding claims, wherein the electrodes (3):

   - are dry electrodes comprising protrusions and/or indentations and/or;
   - comprise one or more metals tolerated by the human skin and/or;
   - comprise conductive fabrics and/or conductive polymers.

10. Device (1) according to any of the preceding claims, wherein the electrodes (3) may be arranged in one or more pluralities of electrodes (3), being said electrodes (3) or said pluralities of electrodes (3) permanently connected with one another or switched sequentially.

11. Device (1) according to any of the preceding claims, wherein the AC generator (4) comprises a monitoring subcircuit (14) comprising an electric sensor (15) adapted to measure the voltage and/or current applied to detect whether the electrodes (3) are in contact with a subject's body.

12. Device (1) according to the preceding claim, wherein the electrodes (3) comprise at least one temperature sensor (16) connected to the monitoring circuit.

13. Device (1) according to any of the preceding claims, wherein the AC generator (4) and the electrodes (3) are embedded in a wearable piece of garment (17).

14. Device (1) according to any of claims 1-11, wherein the AC generator (4) and/or the electrodes (3) are adapted to be implanted in a living body.

15. Device (1) according to any of the preceding claims, further comprising delivery means adapted to deliver a substance into the region encompassed by the electrodes (3) during the suppliance of the AC electric fields.

**Patentansprüche**

1. Vorrichtung (1) zur selektiven Beseitigung und/oder zur Verlangsamung des Fortschreitens von Tumoren (2), umfassend:

   - eine Vielzahl von Elektroden (3); und
   - einen Wechselstromgenerator (4), der elektrisch mit den Elektroden (3) verbunden ist;
   wobei die Elektroden (3) dazu angepasst sind, eine Region (5) des Körpers einer Person zu erfassen, die einen oder mehrere Tumore (2) umfasst;
   wobei der Wechselstromgenerator (4) dazu angepasst ist, eine Vielzahl von elektrischen Wechselstromfeldimpulsen (7) zuzuführen, umfassend:

   - eine Grundfrequenz zwischen 10 kHz und 1 MHz;
   - eine Amplitude zwischen 10 V/cm und 500 V/cm; und
   - Tastverhältnisse zwischen 0,001 % und 10 %;

   und wobei der Wechselstromgenerator (4) ferner dazu angepasst ist, die elektrischen Wechselstromfeldimpulse (7) kontinuierlich über einen Zeitraum von mindestens zwei Stunden zuzuführen.

2. Vorrichtung (1) nach dem vorhergehenden Anspruch, wobei die Dauer der elektrischen Wechselstromfeldimpulse (7) im Wesentlichen gleich oder kürzer als 200 ms ist.

3. Vorrichtung (1) nach einem der vorhergehenden Ansprüche, wobei der Wechselstromgenerator (4) dazu konfiguriert ist, die elektrischen Wechselstromfeldimpulse (7) während zweier oder mehrerer aktiver Phasen zuzuführen, wobei jede der aktiven Phasen von der vorherigen und nächsten aktiven Phase durch eine Zeitperiode getrennt ist, die einer Ruhephase entspricht, in der keine elektrischen Wechselstromfeldimpulse (7) zugeführt werden.

4. Vorrichtung (1) nach dem vorhergehenden Anspruch, wobei jede der aktiven Phasen länger als 2 Stunden und kürzer als 24 Stunden ist, und wobei jede der Ruhephasen länger als 2 Stunden und kürzer als 7 Tage ist.

5. Vorrichtung (1) nach einem der vorhergehenden Ansprüche, wobei die elektrischen Wechselstromfeldimpulse eine sinusförmige Wellenform oder eine Wellenform ohne eine Gleichstromkomponente umfassen.

6. Vorrichtung (1) nach einem der vorhergehenden Ansprüche, wobei der Generator dazu angepasst ist, eine konstante Amplitude von weniger als 10 V/cm der gepulsten Wechselstromfelder zwischen den Bursts (7) aufrechtzuerhalten.

7. Vorrichtung (1) nach einem der vorhergehenden Ansprüche, wobei der Wechselstromgenerator (4) einen Sperrkondensator umfasst, der mit den Elektroden (3) in Reihe geschaltet ist, wobei der Sperrkondensator dazu angepasst ist, den Durchgang eines Gleichstroms zu verhindern und den Fluss eines elektrischen Wechselstroms zu ermöglichen, der den elektrischen Wechselfeldern zugeordnet ist.

**8.** Vorrichtung (1) nach einem der vorhergehenden Ansprüche, ferner umfassend Amplitudenmodulationsmittel, so dass die Amplitude der elektrischen Wechselfelder moduliert werden kann, um einen sanften Start und/oder ein sanftes Ende zu erhalten.

**9.** Vorrichtung (1) nach einem der vorhergehenden Ansprüche, wobei die Elektroden (3):

- Trockenelektroden sind, die Vorsprünge und/oder Vertiefungen umfassen und/oder;
- ein oder mehrere Metalle umfassen, die von der menschlichen Haut toleriert werden und/oder;
- leitfähige Stoffe und/oder leitfähige Polymere umfassen.

**10.** Vorrichtung (1) nach einem der vorhergehenden Ansprüche, wobei die Elektroden (3) in einer oder mehreren Vielzahlen von Elektroden (3) angeordnet sein können, wobei die Elektroden (3) oder die Vielzahlen von Elektroden (3) permanent miteinander verbunden sind oder sequentiell geschaltet werden.

**11.** Vorrichtung (1) nach einem der vorhergehenden Ansprüche, wobei der Wechselstromgenerator (4) eine Überwachungsteilschaltung (14) umfasst, die einen elektrischen Sensor (15) umfasst, der dazu angepasst ist, die angelegte Spannung und/oder den angelegten Strom zu messen, um zu erkennen, ob die Elektroden (3) in Kontakt mit dem Körper einer Person sind.

**12.** Vorrichtung (1) nach dem vorhergehenden Anspruch, wobei die Elektroden (3) mindestens einen Temperatursensor (16) umfassen, der mit der Überwachungsschaltung verbunden ist.

**13.** Vorrichtung (1) nach einem der vorhergehenden Ansprüche, wobei der Wechselstromgenerator (4) und die Elektroden (3) in ein tragbares Kleidungsstück (17) eingebettet sind.

**14.** Vorrichtung (1) nach einem der Ansprüche 1-11, wobei der Wechselstromgenerator (4) und/oder die Elektroden (3) dazu angepasst sind, in einen lebenden Körper implantiert zu werden.

**15.** Vorrichtung (1) nach einem der vorhergehenden Ansprüche, ferner umfassend Abgabemittel, die dazu angepasst sind, eine Substanz in die von den Elektroden (3) erfasste Region während der Zufuhr mit den elektrischen Wechselstromfeldern abzugeben.

**Revendications**

**1.** Dispositif (1) pour éradiquer sélectivement et/ou pour ralentir la progression de tumeurs (2) comprenant :

- une pluralité d'électrodes (3) ; et
- un générateur de CA (4) relié électriquement aux électrodes (3) ;
dans lequel les électrodes (3) sont adaptées pour couvrir une région (5) du corps d'un sujet comprenant un ou plusieurs tumeurs (2) ;
dans lequel le générateur de CA (4) est adapté pour fournir une pluralité de impulsions de champ électrique de CA (7) comprenant :

- une fréquence fondamentale comprise entre 10 kHz et 1 MHz ;
- une amplitude comprise entre 10 V/cm et 500 V/cm ; et
- des rapports cycliques compris entre 0,001 % et 10 % ;

et dans lequel le générateur de CA (4) est adapté en outre pour fournir les impulsions de champ électrique de CA (7) en continu pendant une période d'au moins deux heures.

**2.** Dispositif (1) selon la revendication précédente, dans lequel la durée des impulsions de champ électrique de CA (7) est sensiblement égale ou inférieure à 200 ms.

**3.** Dispositif (1) selon l'une quelconque des revendications précédentes, dans lequel le générateur de CA (4) est configuré pour fournir les impulsions de champ électrique de CA (7) pendant deux ou plusieurs phases actives, chacune desdites phases actives étant séparée de la phase active précédente et suivante par une période de temps correspondant à une phase de repos dans laquelle aucun impulsion de champ électrique de CA (7) n'est fournie.

4. Dispositif (1) selon la revendication précédente, dans lequel chacune des phases actives est supérieure à 2 heures et inférieure à 24 heures, et dans lequel chacune des phases de repos est supérieure à 2 heures et inférieure à 7 jours.

5. Dispositif (1) selon l'une quelconque des revendications précédentes, dans lequel les impulsions de champ électrique de CA comprennent une forme d'onde sinusoïdale ou une forme d'onde sans composante de CC.

6. Dispositif (1) selon l'une quelconque des revendications précédentes, dans lequel le générateur est adapté pour maintenir une amplitude constante inférieure à 10 V/cm des champs de CA pulsés entre les salves (7).

7. Dispositif (1) selon l'une quelconque des revendications précédentes, dans lequel le générateur de CA (4) comprend un condensateur de blocage relié en série aux électrodes (3), ledit condensateur de blocage étant adapté pour empêcher le passage d'un courant CC et pour permettre la circulation d'un courant électrique CA associé aux champs électriques de CA.

8. Dispositif (1) selon l'une quelconque des revendications précédentes, comprenant en outre des moyens de modulation de l'amplitude de telle sorte que l'amplitude des champs électriques de CA peut être modulée afin d'obtenir un début progressif et/ou une fin progressive.

9. Dispositif (1) selon l'une quelconque des revendications précédentes, dans lequel les électrodes (3) :

   - sont des électrodes sèches comprenant des protubérances et/ou des indentations et/ou ;
   - comprennent un ou plusieurs métaux tolérés par la peau humaine et/ou ;
   - comprennent des tissus conducteurs et/ou des polymères conducteurs.

10. Dispositif (1) selon l'une quelconque des revendications précédentes, dans lequel les électrodes (3) peuvent être disposés en une ou plusieurs pluralités d'électrodes (3), lesdites électrodes (3) ou lesdites pluralités d'électrodes (3) étant reliées en permanence les unes aux autres ou commutées séquentiellement.

11. Dispositif (1) selon l'une quelconque des revendications précédentes, dans lequel le générateur de CA (4) comprend un sous-circuit de surveillance (14) comprenant un capteur électrique (15) adapté pour mesurer la tension et/ou le courant appliqués pour détecter si les électrodes (3) sont en contact avec le corps d'un sujet.

12. Dispositif (1) selon la revendication précédente, dans lequel les électrodes (3) comprennent au moins un capteur de température (16) relié au circuit de surveillance.

13. Dispositif (1) selon l'une quelconque des revendications précédentes, dans lequel le générateur de CA (4) et les électrodes (3) sont intégrés dans un vêtement portable (17).

14. Dispositif (1) selon l'une quelconque des revendications 1-11, dans lequel le générateur de CA (4) et/ou les électrodes (3) sont adaptées pour être implantés dans un corps vivant.

15. Dispositif (1) selon l'une quelconque des revendications précédentes, comprenant en outre des moyens d'administration pour administrer une substance dans la région couverte par les électrodes (3) pendant la fourniture des champs électriques de CA.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

**FIG. 15**

**FIG. 16**

**EP 4 541 415 B1**

**Patent documents cited in the description**

- US 2005209641 A